Europäisches Patentamt

European Patent Office

Office européen des brevets

(11) Publication number: **0 459 433 A1**

# EUROPEAN PATENT APPLICATION

(21) Application number: 91108766.6

(22) Date of filing: 29.05.91

(51) Int. Cl.⁵: **B60J 3/04**, G02F 1/13

(30) Priority: 01.06.90 IL 94597

(43) Date of publication of application:
04.12.91 Bulletin 91/49

(84) Designated Contracting States:
DE FR GB IT

(71) Applicant: OFEK A.T. TECHNOLOGIES LTD.
Givat HaMeyasdim, Mishor Adumim, P.O.Box 4530
Jerusalem(IL)

(72) Inventor: Bienenstock, Marc
1, Zeira Street
Jerusalem 93184(IL)

(74) Representative: Quinterno, Giuseppe et al
c/o Jacobacci-Casetta & Perani S.p.A. Via Alfieri, 17
I-10121 Torino(IT)

(54) **Anti-dazzle apparatus.**

(57) An anti-dazzle apparatus comprises:
(a) image-sensing means (3/7);
(b) controllable light-attenuating means (4/5) positioned in front of the eyes of the user; and
(c) control means (12/13) to control the controllable light-attenuating means according to the signal received from the image-sensing means.

The apparatus is useful in avoiding dazzle of the wearer deriving from vehicles and other light sources.

FIG. 1

## Field of The Invention

The present invention relates to an anti-dazzle apparatus. More particularly, the invention relates to an apparatus which is capable of reducing the effect of strong light sources such as the headlights of a motor vehicle.

## BACKGROUND OF THE INVENTION

It is known in the art to provide means for shading excessively strong light. One instance is photosensitive eyeglasses, which become darker in sunlight and in this way protect the eyes of the wearer in a manner which is proportional to the surrounding light. Another approach involves providing anti-glare glasses which consist of a photosensitive layer associated with an optical system. One such type of glasses is described in UK Patent Application GB 2 152 661, which relates to glasses in which the photo-sensitive layer comprises an array of light sensors, each of which controls a sub-area of a liquid crystal cell which constitute the eyeglasses. This type of device has two main disadvantages: (1) Because the liquid crystal is too close to the eye, the protective effect of a small LCD pixel will be averaged out over a large portion of the eye's field of view, providing only little protection at the location where it is needed on the one hand, and strong light attenuation in the surrounding area on the other hand, which is undesirable. Moreover, when only one protective LCD pixel is darkened as a result of the detection by one corresponding detector, the optical size of the two elements, viz. the LCD pixel and the detector, are more or less equivalent, which means that the level of protection which may be achieved against small distant light sources is very low. (2) Because of the short distance from the eyes, the movement of the eyes relatively to the shield is significant, and the protecting spots are not always positioned at the effective location. Glasses of the type described in GB 2 152 661 may be partially effective in shielding against large light sources, but are virtually ineffective in the case of small distant light sources.

Another approach is that followed in US Patent 3,961,181, in which a fixed system is positioned on a vehicle, a shield is placed in front of the driver and detectors are positioned on the car. The disadvantage of this method is that it is impossible to know where the head of the driver is positioned at any given time, and for that reason, the thin strips of the shield which are darkened for protection may prove to be largely uneffective due to head movement. Additionally, the shield according to this patent is darkened only in the shape of vertical strips, thus unnecessarily obscuring significant portions of the field of view for every small light source.

It is an object of the present invention to provide a device which obviates the aforesaid disadvantages, and which can be used effectively to shield the eyes of a driver, or any other person employing it, from strong light sources.

It is another object of the invention to provide a device which is simple and effective in operation, and which does not require darkening large areas in front of the eyes of the user, thus leaving maximal field of view.

## SUMMARY OF THE INVENTION

The anti-dazzle apparatus according to the invention comprises:

(a) image-sensing means;

(b) controllable light-attenuating means positioned in front of the eyes of the user; and

(c) control means to control the controllable light-attenuating means according to the signal received from the image-sensing means.

According to one embodiment of the invention head tracking means are provided, to provide data on the location of the head of the user respective to the shield. In a preferred embodiment of the invention the head tracking means consist of, or are coupled to, the image-sensing means, e.g. a camera, and are attached to the head of the user.

Alternatively, it is possible to provide independent head tracking means located, e.g., on the roof of a vehicle or on the shield itself, which will continuously provide position data to the control means, relative to some initial reference position, in order to adjust the coordinates of the darkened portions of the shield according to the movement of the head.

According to one embodiment of the invention, the image-sensing means comprise a camera, preferably a CCD camera or photodiodes array camera. Suitable cameras of this type are known to the skilled person, and can be custom-made to provide the required characteristics. One example of a suitable CCD camera is a camera with an aperture angle of approximately ±30° in elevation and azimuth. The minimum desirable resolution of the camera would be approximately 30 x 30 pixels. Such a camera may be assembled, e.g., from Monochrome Imaging Module 56474 or 56475 (Philips, Eindoven, Holland), equipped with a suitable lens or a pinhole diaphragm. Such a camera assembled from the Philips module may have a resolution of 604 x 588 pixels, viz., a resolution much higher than required. As will be apparent to the skilled person, simpler, cheaper and lighter cameras can be provided, if desired.

The controllable light-attenuating means may be of any suitable type in which a matrix of dots can be selectively darkened by appropriate control means, or any array of light-switching elements can be selectively switched into a transmissive or blocked mode by appropriate control means. According to a preferred embodiment of the invention the shield comprises a transmissive LCD high-contrast Dot Matrix. Matrices of this type are commercially available (e.g., from Hamlin, Inc., U.S.A.) or can be custom-made according to the specific requirements of the shield. Preparing such a matrix is within the scope of the routineer.

According to one embodiment of the invention, the light-attenuating means are positioned at a distance of at least 50 mm from the eye, as will be explained in detail hereinafter. In another preferred embodiment of the invention, the light-attenuating means may be positioned at a distance shorter than 50 mm from the eye, but this will result in less effective protection for each LCD subarea, as will be explained in detail below. According to this embodiment of the invention, therefore, it is necessary to enlarge the protective LCD areas for each detected dazzling source in order to achieve effective protection. This, of course, will result in a greater portion of the field of view of the user being obscured by darkened areas.

Thus, according to this latter embodiment of the invention, it is possible to adjust the efficiency of protection of the apparatus, according to different distances from the eye and individual preference. This, of course, will also permit to provide apparatus in which the light-attenuating means can be moved at will by the user, according to individual preference at any given time. The enlargement factor of the LCD subareas can be automatically controlled, according to the distance of the light-attenuating means from a reference point, or may be manually controlled by the user by means of an appropriate manual control.

As said, the controllable light-attenuating means (referred to hereinafter as "shield", for the sake of brevity) should be positioned at a distance of at least 50 mm from the eye, and preferably at least 70 mm, when no enlargement capability is provided for the darkened subareas, and in any case when optimal protection is to be achieved. This is needed in order for the shield to function as a "field stop". The meaning of "field stop" is well known in the art (e.g., from Jenkins & White, Fundamentals of Optics, 4th Ed., McGraw-Hill, Ch. 7, pp. 115 - 117).

Broadly speaking, a "field stop" is a stop that can selectively determine the extent of the field which will be present in the image (as opposed to an "aperture stop" which is achieved when the stop is close to the lens and controls the general brightness of the field of view).

If the shield is located at about 150 mm from the eyes of the user, a small obscured area of 3 mm in the shield will be sufficient to mask a light source of 2 m in diameter located at a distance of 100 m from the user. The light source will be virtually entirely obscured, while taking the toll of only about 1.2° off the whole field of view of the user.

If, on the other hand, the shield were positioned at a distance of only 15 mm from the eye, then an area of 3.5 mm in diameter would mask the same light source with only 50% efficacy, but this obscured area would mask an aperture angle of about 13° of the field of view of the user.

The control means should be adapted to control the controllable light-attenuating means by elaborating the signal received from the image-sensing means, which must sense spots of bright light. Such control means preferably comprise a microprocessor or the like device for elaborating and transmitting the signal. One instance of a suitable microprocessor is the general purpose microprocessor 8051, manufactured by Intel. If head-tracking means are provided, then the signals originating therefrom are also elaborated by the control means.

In addition to the above-mentioned operations, the control means must "decide" whether a dazzling condition exists, before it controls the darkening of a given area. For this purpose background lighting detection means must be provided, to obtain and feed to the control means data on the background lighting which can be compared with the data on the bright spots detected by the image-sensing means. Appropriate settings will be provided in the control means in order to effect the darkening of a given shield zone as a result of the comparison of relative intensity data. Background lighting measurements may be obtained either from the interpretation of the image-sensing means signals, or from an additional photodetector.

Furthermore, the control unit may be programmed so as to darken duplicate protective spots in front of one eye as a result of required spots for the protection of the second eye. These duplicate spots, being small in size, will not significantly affect the field of view of the eye in front of which they appear.

If the image-sensing means are coupled to the head of the user, the shield may be broadly positioned in two different positions: (1) on the head of the user, in structural combination with appropriate head-supporting means as well as with the control means; or (2) the shield may be located elsewhere, e.g., may be attached to the vehicle, and may be connected to the controller and the image-sensing means positioned on the head of the user. In this case, the image sensing means may provide to the control means the information on the location of the head of the driver relative to the shield, e.g. by monitoring the location of reference LEDs appropriately mounted on the shield and oriented toward the image-sensing means.

## Brief Description of the Drawings

All the above and other characteristics and advantages of the invention will be better understood from the following illustrative and non-limitative description of preferred embodiments, with reference to the appended drawings, wherein:
- Fig. 1 is a schematic view of a device according to one embodiment of the invention;
- Fig. 2 is a schematic view of a device according to another embodiment of the invention;
- Fig. 3 illustrates the operation of the apparatus according to the invention; and
- Fig. 4 is a schematic representation of the optical functioning of the apparatus.

## DETAILED DESCRIPTION OF PREFERRED EMBODIMENTS

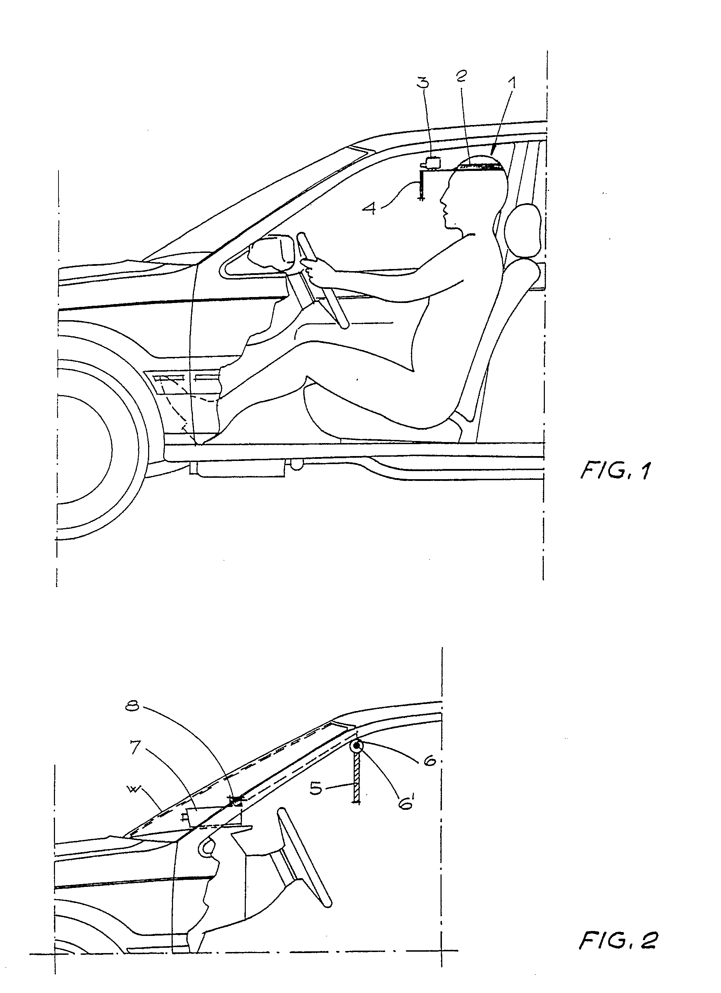

Referring now to Fig. 1, the user, which may be a driver, is using a helmet-type apparatus, generally indicated by numeral 1. This apparatus comprises head-supporting means 2, a CCD camera 3, and a shield 4, which comprises a transmissive liquid crystal device. The control means (not seen in the figure) connects between the CCD camera 3 and the shield 4, and are operated by appropriate power sources (not shown), e.g.,internal batteries or the vehicle battery.

In Fig. 2 a device is seen in which the shield is not positioned on the head of the user, but may be, e.g., fixed on a vehicle. In this case the shield 5 is connected to a support 6, which can be designed to permit to flip up the shield 5, e.g., by rotating around an axis 6', or by any other suitable arrangement. The shield 5 and the CCD camera 7, positioned in front of the windshield, W, are connected to the controller, 8.

According to this embodiment of the invention, if no head-tracking device is provided, the protective areas may be enlarged to compensate for uncontrolled head movement. Enlargement of the protective areas may be effected, for instance, by the user himself, by means of a manual control.

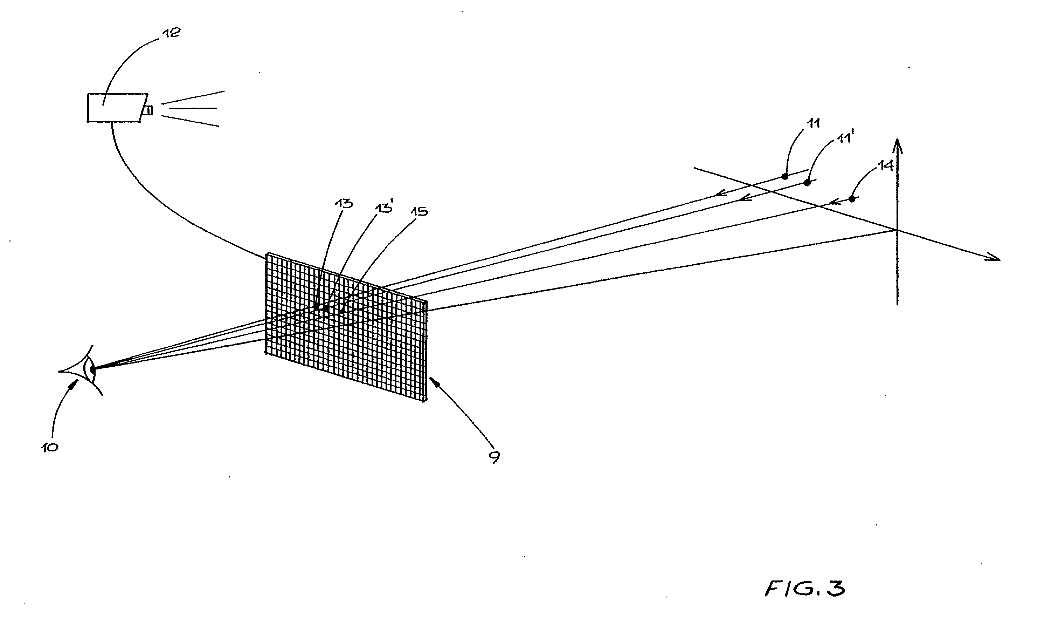

Fig. 3 illustrates the operation of the device of the invention, regardless of whether this is a head unit as in Fig. 1, or a fixed unit, as in Fig. 2. The shield 9 consists of a plurality of independently darkenable areas (not shown individually in the figure, for the sake of simplicity). The eye of the viewer, indicated by numeral 10, is positioned at a distance which is at least 50 mm from the shield.

When lights enter the field of view of the user, which lights may be, e.g., the headlights 11 and 11' of a motorcar coming in front of the user, the camera, 12, will identify them and will transmit through a controller (not seen in the figure) an order to the screen to darken the relevant LCD areas 13 and 13'. If a low-intensity object, 14, remains in the field of view, then the corresponding LCD area 15 in screen 9 is not darkened, and the object is seen in much better contrast since the eye of the viewer is shielded from lights 11 and 11'.

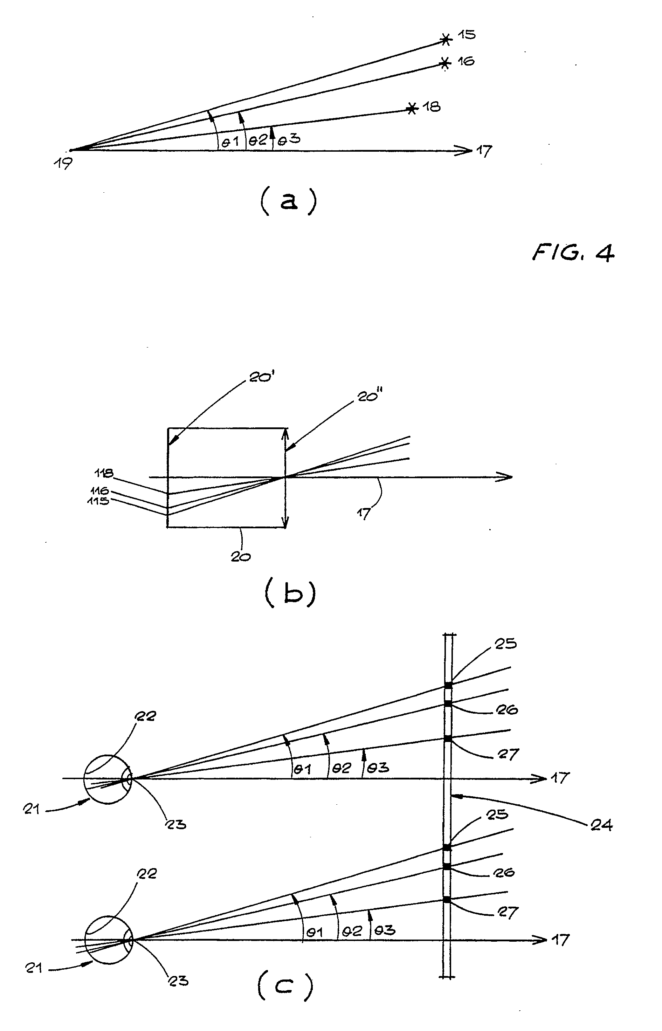

Fig. 4 is a schematic presentation of the optical functioning of the apparatus of the invention, regardless of whether fixed on a vehicle or head-worn. In Fig. 4(a) are the two headlights of an oncoming vehicle, 15 and 16, which make an angle with the optical axis 17 of the CCD camera. An additional object, 18, is also shown, and the head of the driver is indicated by numeral 19.

In Fig. 4(b) the image of headlights 15 and 16 of Fig. 4(a) is indicated by numeral 115 and 116 respectively, and that of object 18 by numeral 118. The camera 20 has a sensitive surface, 20', and a front lens, 20''.

In Fig. 4(c) the two eyes of the user are seen, indicated by 21; for each eye the retina 22 and the pupil 23 are shown. The shield 24 is placed in front of the eyes 21.

In the situation shown in Fig. 4, two protective LCD areas 25 and 26 are shown, which will shield the eyes from headlights 15 and 16 (Fig. 4(a)), while viewable object 18 will be in front of the transmissive LCD area 27. The rest of the shield, of course, will also be transmissive at all areas which are not in front of additional lights.

Shield 24 is preferably positioned at a distance greater than four focal lengths (> 68 mm) from the eye, and therefore it has a real image which is substantially well focused on the retina of the eye. Consequently, areas 25 and 26 are reasonably well imaged on the retina and thus effectively mask the corresponding real images of light sources 15 and 16.

Each distant light source is imaged by the front lens 20'' of the camera 20 (Fig. 4(b)) on the sensitive surface 20' of the camera, in the same way that it is imaged on the retina of the eye. The control means is programmed to darken such an area of the shield, in front of each eye, which will be imaged on the retina of the eye at the same place where the light source is imaged.

As will be apparent to the skilled person, the darker the areas 25 and 26 of the shield, the more light sources 15 and 16 will be neutralized. Therefore it will be generally desirable to employ a high-contrast shield. It is of course possible to darken each area independently in varying degrees, e.g.,

depending on the intensity of the light sources 15 and 16.

Additionally, since the eyes are generally more comfortable with not-too-sharp darkness transitions, the LCD areas of the shield surrounding a fully darkened area may be brought to an intermediate level of shade. This smoothening of the darkness transitions is automatically achieved by a limited out-of-focus condition, prevailing when the shield is not too far away from the eyes.

As will be apparent to the skilled person, when the apparatus is helmet-mounted as in Fig. 1, since the camera is coupled to the head of the user, it sees the same lights as the eye sees, even if the head of the user moves, as it normally does, particularly if the user is a driver. Thus the dazzling sources are covered and attenuated regardless of the movement of the driver. This means for instance that the darkened, protective zones of the shield are changed and moved as the head of the user rotates, in order constantly to shield the eyes of the driver regardless of the movements of his head relative to the light sources.

In the case of a fixed-type apparatus, such as that shown in Fig. 2, the protective zones of the shield are obscured at such locations which will individually shield the eyes of the driver as appears from Figs. 3 and 4. If suitable head-tracking means are provided, then the protective zones can be automatically moved by the control means in accordance with the movement of the head of the driver. If no tracking means are provided, then the protective zones may be somewhat enlarged in order to compensate limited movements of the head of the user.

As will be apparent, it may be desirable to provide means of calibrating the apparatus of the invention. Calibration procedure may include, e.g., the compensation of different spacing between the eyes of different drivers and the visual height of the LCD screen, all of which can be different from one driver to another and may also change from time to time, depending on how the apparatus is worn, in the case of a helmet-type apparatus, and of how the driver is seated in the case of a fixed-type apparatus. Calibration may be performed, e.g. by the operator who will adjust the location of the protective spots while looking at a fixed bright light using an electronic input of the control means.

As said, it is further possible to provide intermediate gray shades for each protective area, so as to smooth transition effects. In the case of fast-moving light sources the next location of the source may be extrapolated from the past movement. It is likewise possible to provide many optional features, such as different settings for different drivers, the driving conditions being memorized for each driver. Correction for different parameters, such as strong glasses worn by the driver, may also be provided.

It should further be noted that the apparatus of the invention, while being particularly convenient for drivers, is not limited to driving activity. It may be employed for looking at bright scenes in general, in which relatively dark objects are found and are to be observed. For instance, ships on brightly illuminated seas, airplanes appearing against bright skies, etc. The apparatus of the invention may automatically darken the overall scene for the observers, while leaving the viewing path to the dark object fully transmitted. Many other changes and uses will be understood by the skilled person, and can be exploited without exceeding the scope of the invention. For instance, the apparatus of the invention may be exploited also to shield the light reflected on the rear-view mirror, thus avoiding the need for separate anti-dazzling means.

## Claims

1. Anti-dazzle apparatus, comprising:
   (a) image-sensing means;
   (b) controllable light-attenuating means positioned in front of the eyes of the user; and
   (c) control means to control the controllable light-attenuating means according to the signal received from the image-sensing means.

2. Apparatus according to claim 1 wherein the light-attenuating means are positioned at a distance of at least 50 mm from the eye.

3. Apparatus according to claim 1, wherein the light-attenuating means are positioned at a distance of 50 mm or less from the eye, and enlargement means are provided for automatically or manually enlarging the darkened subareas of the light-attenuating means as a result of the reduced distance from the eyes.

4. Apparatus according to claim 1 to 3, wherein the image-sensing means comprise a camera.

5. Apparatus according to claim 4, wherein the camera is a CCD camera.

6. Apparatus according to any one of claims 1 to 5, wherein the controllable light-attenuating means comprise a shield consisting of a plurality of individually darkenable subareas.

7. Apparatus according to claim 6, wherein the shield is a liquid crystal display.

8. Apparatus according to any one of claims 1 to 7, wherein the control means comprise a microprocessor or the like signal-elaborating device.

9. Apparatus according to any one of claims 1 to 8, comprising, in structural combination, head-supporting means, image-sensing means, controllable light-attenuating means and control means.

10. Apparatus according to any one of claims 1 to 8, wherein the controllable light-attenuating means are supported on a vehicle or other frame.

11. Apparatus according to claim 10, further comprising head-tracking means to provide data on the relative position of the head of the user.

12. Apparatus according to claim 11, wherein the head-tracking means consist of, or are coupled to, the image-sensing means.

13. Apparatus according to any one of the preceding claims, further provided with a background lighting detector to provide data as to the intensity of the lighting of the background.

FIG. 1

FIG. 2

FIG.3

(a)

FIG. 4

(b)

(c)

European
Patent Office

**EUROPEAN SEARCH
REPORT**

Application Number

**EP 91 10 8766**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 848 890 (HORN)<br>* the whole document * | 1,3-9 | B 60 J 3/04<br>G 02 F 1/13 |
| X | PATENT ABSTRACTS OF JAPAN vol. 12, no. 236 (M-715)(3083) 7 June 1988,<br>& JP-A-63 28721 (NIPPON DENSO CO LTD) 6 February 1988,<br>* the whole document * | 1,7,8,9,10 | |
| D,X,D,Y | US-A-3 961 181 (GOLDEN)<br>* abstract; figure 1 * | 1,6,7,10, 11,12 | |
| Y | DE-A-2 158 951 (LICENTIA PATENT-VERWALTUNGS.GMBH)<br>* page 2, lines 7 - 18; figure 1 * | 11,12 | |

TECHNICAL FIELDS
SEARCHED (Int. Cl.5)

B 60 J
G 02 F

The present search report has been drawn up for all claims

| Place of search | Date of completion of search | Examiner |
|---|---|---|
| The Hague | 17 September 91 | FOGLIA A. |